# EUROPÄISCHE PATENTANMELDUNG

(11) **EP 2 226 628 A1**
(43) Veröffentlichungstag der Anmeldung: **08.09.2010**
(21) Anmeldenummer: 10005986.4
(22) Anmeldetag: 02.10.2008
(51) Int. Cl.: G01N 27/407

(54) **Gassensor**

(30) Priorität: 05.10.2007 DE 102007048049
(62) Teilanmeldung aus: 08837966.4
(71) Anmelder: HERAEUS SENSOR TECHNOLOGY GMBH, D-63450 Hanau (DE); J. Dittrich Elektronic GmbH & Co.KG,, 76532 Baden-Baden (DE)
(72) Erfinder: Turwitt, Martin, 63486 Bruchköbel (DE); Wienand, Karlheinz, 63741 Aschaffenburg (DE); Ullrich, Karlheinz, 64823 Groß-Umstadt (DE); Asmus, Tim, 35469 Allendorf-Winnen (DE); Dittrich, Jürgen, 76532 Baden-Baden (DE); Schönauer, Ulrich, 76344 Eggenstein (DE); Guth, Ulrich, 04720 Ziegra-Knobelsdorf (DE)
(74) Vertreter: Kühn, Hans-Christian

(57) **Zusammenfassung**

Ein einziger lonenleiter wird zur Messung von die Umwelt belastenden Abgaskomponenten verwendet, insbesondere als Sauerstoffpumpe. Zur Bestimmung von NOₓ im Abgas wird ein Sensorchip im Abgaskanal montiert wird, der einen einzigen lonenleiter zur Bestimmung von NOₓ aufweist. Vorzugsweise weist der Sensorchip einen Heizwiderstand auf und wird mit diesem geheizt. Ein Chip mit Heizleiter und Sauerstoffpumpe auf dessen Substrat ist mit einem Abgasschutz ausgestattet. Die äußere Elektrode einer Sauerstoffpumpe enthält neben Platin mindestens 20 Gew.-% Gold, Rhodium, Ruthenium, Palladium oder Silber. Auf einer elektrisch isolierenden Oberfläche eines Substrats ist eine Widerstandsstruktur und parallel hierzu beabstandet ein lonenleiter angeordnet, der als Deckel einen Raum zwischen dem Substrat und Seitenwänden abschließt.

## Beschreibung

Die vorliegende Erfindung betrifft die Messung von unerwünschten Gasen im Abgas von Verbrennungsmaschinen oder Verbrennungsanlagen, eine diesbezügliche Verwendung von Sauerstoffpumpen und geeignete Sensoren.

Gemäß DE 196 51 328 B4 basieren Sauerstoffpumpen auf einer elektrochemischen Reaktion von Zirkoniumdioxid zwischen zwei Elektroden. Diese Vorrichtung vermag Sauerstoff von einer Seite einer Zirkoniumdioxid-Wand über dessen Reduktion zu Sauerstoffionen an der Kathode, Ionenwanderung der Sauerstoffionen im ZrO₂ und Oxidation der Sauerstoffionen zu Sauerstoff an der Anode zur anderen Seite zu transportieren bzw. zu pumpen. Auf diese Weise ist ein unterschiedlicher Partialdruck an den beiden Seiten erzielbar.

Die Messung unerwünschter Bestandteile von Abgasen, insbesondere deren NOₓ-Gehalt, erfolgt bislang anhand aufwändiger stationärer Analysegeräte. Eine bekannte AuPtRh-IDK-Struktur ist querempfindlich und liefert deshalb in keine eindeutigen Ergebnisse.

Die Aufgabe der vorliegenden Erfindung besteht darin, eine zuverlässige Abgasanalyse, insbesondere NOₓ-Messung, auf einfache Art und Weise in Verbrennungskraftmaschinen oder Verbrennungsanlagen zu integrieren.

Zur Lösung der Aufgabe wird ein Ionenleiter oder eine Sauerstoffpumpe, insbesondere eine einen Hohlraum umschließende Materialpaarung aus Edelmetallelektroden und Zirkoniumdioxid, das vorzugsweise mit Yttriumoxid oder Hafniumoxid stabilisiert ist, in einem begrenzten Temperaturbereich gassensitiv, insbesondere NOₓ-sensitiv für wenigstens eine Umwelt belastende Abgaskomponente verwendet, insbesondere NOₓ.

Die Lösung der Aufgabe erfolgt mit den Merkmalen der unabhängigen Ansprüche. Bevorzugte Ausführungen sind in den abhängigen Ansprüchen beschrieben.

Erfindungsgemäß wird ein Ionenleiter bei der Messung von Gaskomponenten verwendet. Insbesondere ist der Ionenteiter Bestandteil einer Sauerstoffpumpe. Erfindungsgemäß weist der Gassensor bzw. die Sauerstoffpumpe eine zwischen Elektroden angeordnete Membran auf einem Ionenleiter, insbesondere ZrO₂, auf. Erfindungsgemäß ist damit nur noch ein Ionenleiter erforderlich, dessen Elektroden gemeinsam mit einer Vielzahl von "Deckeln" auf einer Platte herstellbar sind, die nach der Erstellung der Elektroden vereinzelt wird. Hierdurch vereinfacht sich die Herstellung eines Gassensors enorm.

Ein maßgebliches Anwendungsgebiet für derartige Sensoren ist die Messung von Abgasbestandteilen. Die Funktionsfähigkeit von Sensoren wird vorzugsweise vor der Beeinträchtigung durch heiße Abgase geschützt, insbesondere durch Anordnung des Sensors in einem gasdurchlässigen Gehäuse. Dies ermöglicht die dauerhafte Bestimmung von NOₓ in Abgasen von Verbrennungskraftmaschinen oder Verbrennungsanlagen. Erfindungsgemäße Sensoren können direkt im Abgaskanal montiert werden.

Hierzu wird ein gassensitiver, insbesondere NOₓ-sensitiver Sensor bereitgestellt, der einen lonenleiter oder eine Sauerstoffpumpe enthält, deren äußere Edelmetallelektrode neben Platin erfindungsgemäß mindestens 20 Gew.-%, insbesondere mindestens 30 Gew.-% eines weiteren Edelmetalls aufweist. Das weitere Edelmetall wird aus der Gruppe bestehend aus Gold, Silber, Palladium, Rhodium und Ruthenium ausgewählt. Insbesondere besteht zumindest ein weiteres Edelmetall aus den Elementen Gold, Silber oder Ruthenium. Der Platinanteil beträgt mindestens 5 Gew.-%, insbesondere 10 bis 30 Gew.-%. Bewährt hat sich eine goldbasierte Legierung mit 10 bis 30 Gew.-% Platin und 5 bis 15 Gew.-% Rhodium.

Eine elektrochemische Sauerstoffpumpe weist zwischen der zu untersuchenden Atmosphäre und einem davon abgetrennten Raum einen Sauerstoffionenleiter auf, der durch eine Potenzialdifferenz zwischen zwei Elektroden gasförmigen Sauerstoff an der Kathode reduziert und Sauerstoffionen an der Anode oxidiert. Hierzu befindet sich eine Platinelektrode in dem geschlossenen Raum. Die äußere Elektrode wird durch zusätzliche Edelmetalle neben Platin in bestimmten Temperaturbereichen gassensitiv für von Sauerstoff verschiedene Gase, insbesondere im Abgas unerwünschte Bestandteile, wie NOₓ.

In erfinderischer Weiterbildung ist ein Gassensor als Chip ausgebildet. Hierzu werden poröse Elektroden auf einem Sauerstoffionenleitersubstrat wie ZrO₂ angeordnet, insbesondere in Dickschicht-Technik. In einer Ausführung ist das Sauerstoff-Leitersubstrat z. B. mit Y₂O₃- oder HfO₂-stabilisiertem ZrO₂ auf einem keramischen Substrat, z. B. Al₂O₃ an seinen Rändern angeklebt, z. B. mit Glas, so dass zwischen dem keramischen Substrat und dem Sauerstoffionenleiter und der Umrandung, z. B. aus Glas, ein Hohlraum gebildet ist. Damit wird erfindungsgemäß ein sensitiver Chip für unerwünschte Abgaskomponenten bereitgestellt, insbesondere zur Bestimmung von NOₓ. Der Chip weist vorzugsweise einen Heizwiderstand auf.

Mit diesem Heizwiderstand lässt sich die Betriebstemperatur des Sensors einstellen und somit die Sensitivität, insbesondere bezüglich NOₓ, optimieren. Der Heizwiderstand ist auf einem elektrisch isolierenden Substrat, insbesondere hochreinem Aluminiumoxid, angeordnet. Der Heizwiderstand wird vorzugsweise in Dünnschichttechnik erzeugt. Die Elektroden, zwischen denen der Ionenleiter, insbesondere ein Zirkoniumdioxid-Plättchen, angeordnet ist, werden mit einem bekannten Verfahren zur Herstellung poröser Elektroden hergestellt, z. B. in Dickschichttechnik oder nach besonders speziellen Dünnschichttechnikverfahren. Vorzugsweise wird der Chip als SMD- oder Flip-Chip-Bauteil bereitgestellt. Die Abmessungen eines solchen Chips, bestehend aus dem elektrisch isolierenden Substrat, insbesondere einem keramischen Substrat mit darauf angeordneter Sauerstoffpumpe, betragen weniger als 5 mm. Der zwischen dem keramischen Substrat und dem Sauerstoffionenleiter befindliche Hohlraum beträgt vorzugsweise unter 1 mm³, insbesondere unter 1/100 mm³.

In bevorzugter Ausführung weist der gassensitive Chip einen Temperatursensor auf. Ein auf dem keramischen Substrat aufgeklebtes ZrO₂-Plättchen, insbesondere mit Y₂O₃-stabilisiertem ZrO₂, welches durch eine Umrandung mit dem Klebemittel vom Keramikplättchen beabstandet ist, hat sich als Sauerstoffpumpe des Chips bewährt. Das Zirkoniumdioxid-Plättchen weist auf einer Seite eine in dem Hohlraum angeordnete Elektrode aus Edelmetall, insbesondere Platin, auf und eine äußere Elektrode, die neben Platin wenigstens ein weiteres Edelmetall aufweist, insbesondere Gold. Bewährt hat sich eine Elektrode aus einer Goldlegierung, die neben 10 bis 30 Gew.-% Platin noch 5 bis 15 Gew.-% Rhodium aufweist.

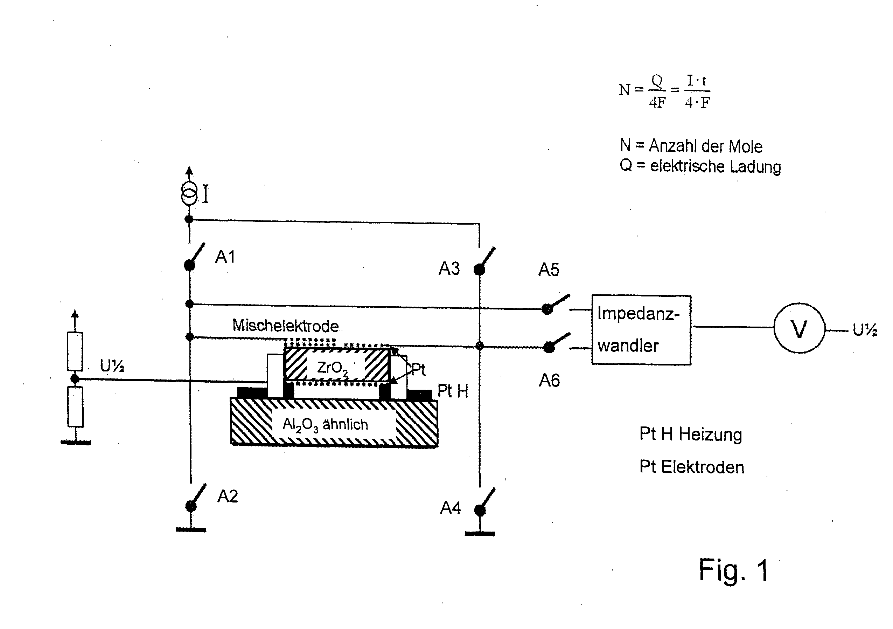

Im Folgenden wird die Erfindung anhand von Beispielen mit Bezug auf die Zeichnungen verdeutlicht. Die Basis ist ein Substrat mit elektrisch isolierender Oberfläche, wie z. B. in Figur 1 ein Plättchen aus Aluminiumoxid. Das Plättchen ist mit einem Heizwiderstand beschichtet, der in Dünnschichttechnik aufgetragen werden kann. Der Heizwiderstand ist hier außerhalb von Wänden angeordnet, die mit einem Deckel aus einem Ionenleiter, hier Zirkoniumdioxid, und der Substratoberfläche einen Raum abschließen. Ober- und unterhalb des Plättchens aus Zirkoniumdioxid sind metallisch leitende Elektroden angeordnet, von denen in Figur 1 zwei als Platinelektroden ausgeführt sind. Die als Mischelektrode bezeichnete Elektrode besteht aus einer Edelmetalllegierung, die Gold und Platin aufweist. Die Elektroden sind so porös, dass sie sauerstoffdurchlässig sind. Hierzu werden sie vorzugsweise per Siebdruck aufgetragen.

Zur Herstellung in Massenproduktion wird eine Vielzahl von Zirkoniumplättchen gemeinsam erzeugt, indem ein großes Substrat aus Zirkoniumdioxid entsprechend mit Elektroden strukturiert wird, um dann vereinzelt zu werden und auf oder zwischen Wänden auf dem Substrat befestigt zu werden. Der Sensor kann dann zur Messung gemäß Figur 1 verschaltet werden.

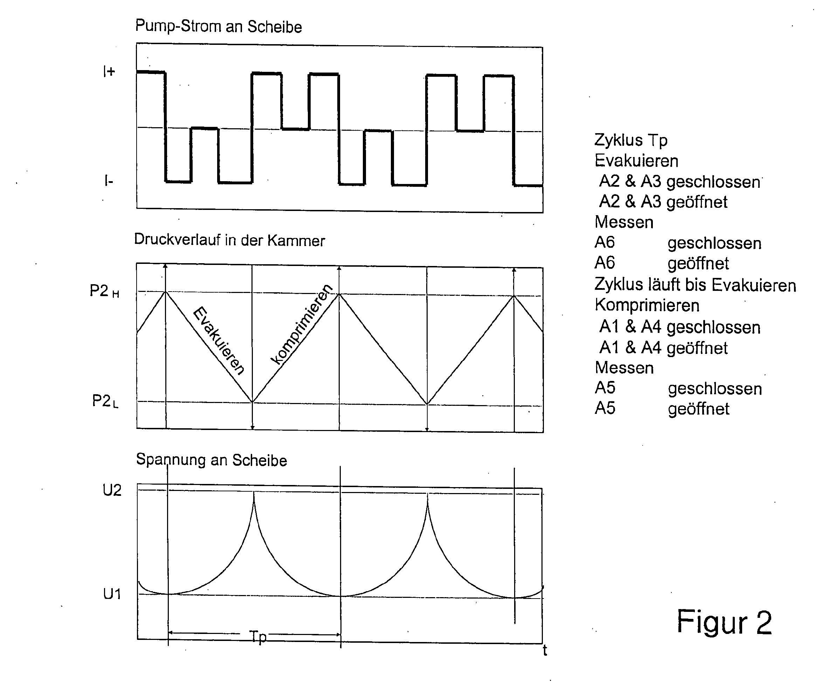

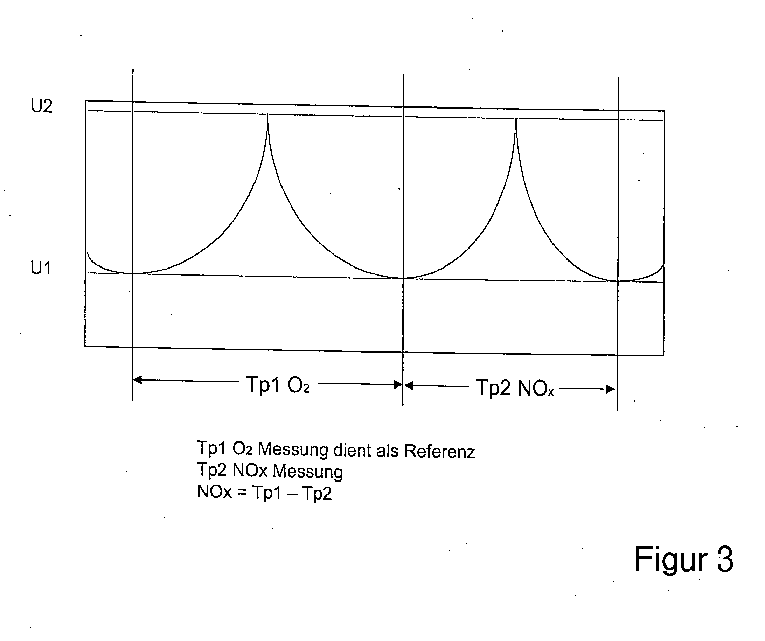

Figuren 2 und 3 zeigen Messzyklen in Bezug auf die elektrischen Anschlüsse und die Druckveränderung in dem eingeschlossenen Raum.

## Patentansprüche

1. Abgassensitiver Chip, **dadurch gekennzeichnet, dass** auf einem keramischen Substrat ein Heizleiter und eine Sauerstoffpumpe zur Bestimmung schädlicher Abgaskomponenten angeordnet sind und mit einem Schutz ausgestattet sind, der die Funktionsfähigkeit des Sensors vor der Beeinträchtigung durch heiße Abgase schützt.

2. Gassensor, insbesondere Chip nach Anspruch 1, enthaltend eine Sauerstoffpumpe, die **dadurch gekennzeichnet ist, dass** neben Platin mindestens 20 Gew.-% des Edelmetalls der äußeren Elektrode der Sauerstoffpumpe aus der Gruppe bestehend aus Gold, Rhodium, Ruthenium, Palladium und Silber ausgewählt sind.

3. Gassensor nach Anspruch 1 oder 2, **dadurch gekennzeichnet, dass** die Sauerstoffpumpe eine zwischen Elektroden angeordnete Membran aus ZrO₂ aufweist.
